# EUROPEAN PATENT APPLICATION

(11) **EP 4 285 982 A1**
(43) Date of publication of application: **06.12.2023**
(21) Application number: 22177290.8
(22) Date of filing: 03.06.2022
(51) Int. Cl.: A61M 37/00, A61B 17/06, A61B 17/00

(54) **FELTING SET FOR ATTACHING IMPLANTS TO SOFT TISSUE**

(71) Applicant: ZuriMED Technologies AG, 8008 Zürich (CH)
(72) Inventor: MÖHL, Andrea Gabriela, 5600 Lenzburg (CH); CAMICHEL, Cherilyn Amber, 8706 Meilen (CH); LI, Xiang, 8610 Uster (CH); BACHMANN, Elias, 8049 Zürich (CH)
(74) Representative: Vossius & Partner Patentanwälte Rechtsanwälte mbB

(57) **Abstract**

A felting set (1) for attaching an implant (10) to soft tissue (2). The set comprises a felting device (20) and the implant, wherein the implant comprises a patch comprising a non-woven material made of individual fibers (11), wherein the fibers have a length of 20 to 80 mm, wherein the patch comprises a drug (12). The felting device includes a needle (21) with at least one blade (22) and with at least one barb (23; 24) for delivering the fibers of the patch into the soft tissue.

## Description

The present application relates to a felting set for attaching implants to soft tissue with a felting device and a patch.

The present applicant recently developed a surgical felting device allowing a biomechanically advantageous implantation and attachment of implants to a soft tissue of a patient. The developed device allows an improved fixation as compared to conventional suturing techniques. One example of such a surgical felting device is disclosed in PCT/CH2019/000015. The surgical felting device comprises a needle that repeatedly moves through a surgical felt and into tissue. By embedding strands of a felt inside tissue, the needle creates a strong and well distributed mechanical bond between the felt and the tissue. Compared to conventional suturing, this technique is faster and alleviates adverse effects such as "cheesewiring" of suture, where the suture cuts through tissue which can happen through local stress peaks.

However, while surgical felting a strong mechanical connection and a well distributed mechanical bond, the patch is limited in its applicability. Further, the operation, in which the patch is applied may require additional steps to ensure improved compatibility, healing or other functions. This may require the application of drugs prior (or after) the felting of the patch, which is cumbersome and may lead to a distribution of an applied drug to undesired areas.

EP 2 818 159 A1 shows one option for drug delivery and aims to improve drug delivery EP 2 818 159 A1 discloses a device having a positioning frame which can be adhesively bonded to the skin and to a transdermal therapeutic system which can be inserted into a recess penetrating the positioning frame. The device includes a tool unit that comprises 90 needles with a length of 2,5 mm. The needles may penetrate a top layer of the skin in the recess. In addition, the device includes a drug unit that may also be applied in the recess. However, the mechanical connection shown in EP 2 818 159 A1 is rather weak.

It is the objective technical problem to overcome the above disadvantages of the prior art. In particular, it is the objective technical problem to provide a surgical felting device with enhanced capabilities.

The objective technical problem is solved by the features of the independent claim.

One aspect of the present invention relates to a felting set for attaching an implant to soft tissue. The felting set may be a medicinal device and particularly adapted for mammals, preferably humans. The felting set comprises a felting device and the implant. The implant comprises a patch with a nonwoven material made of individual fibers. The fibers may have a length of 20 to 80 mm and the patch comprises a drug. The felting device includes the needle with at least one blade and at least one barb for delivering the fibers of the patch patched into the soft tissue. The felting device and the needle are described in detail in the applications PCT/CH2019/000015, PCT/EP2020/081887, PCT/EP2020/081891, and PCT/EP2020/081881 filed by the present applicant.

Felting as used herein may be understood as an intertwining and/or entangling of tissue fibers and implant fibers. The device may comprise at least one felting needle that is configured to move reciprocally. A felting needle may be a needle with one or more barbs. The barbs may be configured to push individual fibers of a patch material inwards when the needle is pushed through the implant and into the soft tissue. The reciprocal motion may be understood as a back-and-forth motion. For example, the needle is moved along a line or rotated around an axis about an angle. The implant may be provided with the surgical device and/or may include a fibrous implant material that is suitable to be felted to soft tissue. Implants can also be provided separately from the surgical device. Example materials and example implants are shown for example in PCT/CH2019/000015, PCT/EP2020/081887, PCT/EP2020/081891, and PCT/EP2020/081881.

In one embodiment, the fibers of the patch may comprise the drug. For example, the fibers may be made of or incorporate the drug. The drug may be ad- or absorbed by the fibers. The drug may be solid and form part of the fiber. Thereby, a drug may be delivered directly into the soft tissue leading to a targeted delivery of the drug.

The fibers may be monofilament or multi-filament fibers. The drug may be arranged between and/or around the filaments of the fibers, e.g. soaking, spraying, or powdering the drug on the fibers. Multifilament fibers are particulary useful as the drug-loading may be increased.

In one embodiment, the fibers are polymer fibers and comprise a hybrid structure of drug and polymer. For example the fiber may be made of PGA (Polyglycolide or poly(glycolic acid)) and the hybrid structure may be a PGA-drug conjugation.

In one embodiment the felting set comprises a first and a second layer. The first and the second layer may differ in degradation rate, drug releasing speed, thickness and/or strength. The felting set may also comprise one or more further layers that may also vary in degradation rate, drug releasing speeds, thickness and/or strength. For example, the felting set may comprise a third and/or fourth and/or fifth layer with a degradation rate, drug releasing speed, thickness and/or strength different from the first and/or second layer.

In particular, the first layer may be intended to be in contact with the soft tissue or closer to the soft tissue and may have a higher drug releasing speed than the second layer. Such embodiments may be particularly useful for drugs that effect the implantation, e.g. antibacterials, antibiotics, anticoagulants, anti-inflammatories, muscle relaxants, and sedatives. The drug release may be modified through an appopriate material choice, layer thickness or a coating.

The second layer may have a lower drug releaseing speed than the first layer. This may be particularly useful for drugs that support the healing process, such as growth factors.

In particular, the first layer may be intended to be in contact with the soft tissue or closer to the soft tissue and have a higher degradation rate than the second layer. In some examples only the first layer may be biodegradable. Thereby, a mechanical connection between the patch and the soft tissue may be maintained. If the layer next to the soft tissue remains non or less biodegradable, a tighter interface may be sustained.

In other embodiments the second layer may have a higher degradation rate in soft tissue than the first layer. The second layer may be arranged on a side of the first layer opposing the soft tissue. During felting, at least initially, a larger portion of the fibers of the second layer are felted into the soft tissue. A higher degradation rate of the second layer may result in a more durable connection between the patch and the soft tissue. During felting the needle may tend to felt more of the of fibers of the top layer. Thus, if the top layer is less biodegradable, the connection may be more durable.

Degradation rate as used herein may be understood as disintegration of the fibers in the respective layer.

The first layer may have a higher tensile strength than the second layer. In particular, the fibers of the first layer may have higher tensile strength than the fibers of the second layer. Alternatively or additionally the first layer may include a higher density of fibers. The second layer may be arranged on a side of the first layer opposing the soft tissue. Thereby, shear movement between the soft tissue and the patch is prevented.

The first and second layer may each or individually comprise a drug. If both, the first and second layer comprise a drug, the drugs may have different release speeds rates. Thereby the patch may be loaded with drugs that are released over different periods of time.

The at least one needle may comprise a needle tip. The needle tip is at a radially outer edge and the blade extends across a cross-section of needle to an opposing other radially outer edge. The needle tip may be understood as the most distal part of the at least one needle. Blade may be understood as the portion of a needle with an edge that is designed to puncture the soft tissue. The blade may be straight. The blade may be angled with respect the longitudinal axis. The angle between the blade and the longitudinal axis may be acute. Parts of the blade may be suitable to puncture the soft tissue. The blade may comprise one, two, three or more barbs. The barb(s) may be formed by gap(s) in the blade. The gap(s) may be formed as a slit. The gap(s) may be formed such that a fiber of the patch can be pushed into the soft tissue by the gaps in the needle. The slits may extend substantially along an axis of the needle or perpendicular to the needle or any direction in between. The edge has the advantage of allowing a lower needle penetration depth, since the gaps may be arranged closer to the tip forming barbs that are closer to the tip.

The drug may be held in the space between the fibers. This embodiment in particularly useful for liquid drugs. Further, the felting motion of the needle then directly delivers the drug to the soft tissue. In some embodiments, the drug (or an encapsulation of the drug) may attach to the needle (e.g. through Van der Waals forces) and thereby be transported to the soft tissue. In other embodiments, the drug may be pushed directly by the needle.

In one embodiment, the drug may be attached to the fibers of the patch. These fibers can then be pushed into the soft tissue delivering the drug to the soft tissue.

In a further embodiment, the patch may comprise carrier fibers and drug-loaded fibers. Thereby, the mechanical stability can be improved, in particular by using mechanically strong (e.g. high tensile strength) fibers, while at the same time allowing for a high drug loading, e.g. by using fibers with a lower thickness than the carrier fibers or by using a material that adsorbs or absorbs the drug.

In one embodiment, the the carrier fibres and drug-loaded fibres are dimensioned such that the at least one barbs felts only the drug-loaded fibers into the soft tissue. For example the carrier fibres may have a higher thickness than the drug-loaded fibres. In one example, the barb diameter or gap size may be smaller than the carrier fibers. Additionally or alternatively, the barb diameter or gap size may be larger than the the drug-loaded fibers. Thereby, the needle can catch the drug loaded fibres and move the carrier fibres aside without pulling them into the soft tissue. It should be noted that the fibres with the higher thickness do not need to be drug loaded.

The fibres may consist of or comprise PET, PLA, PGA, HA (hyaluronic acid), PTFE, PCL (Polycaprolactone), and/or PGA.

In one embodiment, the patch comprises liposomes or micelles, wherein the liposomes or micelles carry the drug. Liposomes and micelles are particularly suitable since they enable a targeted release. The at least one needle can pierce the liposomes and micelles, thereby releasing the drug. In alternative embodiments, the drug release by may be triggered either by internal stimuli (biomarker, pH, redox) or by external ones such as temperature, light, radiation, or ultrasound as is known in the art.

The needle is shaped such that the liposomes or micelles are damaged such that the drug is released when the sharp needle dampges the liposomes while felting the patch. In particular, the needle may comprise a sharp tip. Needles as described herein are particularly useful for releasing compounds such as drugs held in micelles or liposomes mechanically.

In one embodiment, the drug is held by a carrier, in particular nanocapsules or nanospheres, wherein the carrier is loaded onto the fibres of the patch. Nanocapsules or nanospheres are particularly useful since they may allow for a sustained drug release over time in a controlled manner. The nanocapsules may hold the drug and attach to the fibres of the patch.

In one embodiment, the at least one barb has a depth of 0.005-0.1 mm, preferably 0.01-0.05 mm. These depths are particularly suitable for fibrous patches and for pushing individual fibers. Smaller barbs will be able to catch smaller fibers. If the fiber is larger than the barb, the fiber will not be caught.

In one embodiment the needle has a diameter of 0.1 to 0.5 mm. The largest section needle comprising the blade may have a diameter of 0.1 to 2 mm, preferably 0.1 to 1 mm, particularly preferred 0.1 to 0.5 mm.

In one embodiment, the section of the needle comprising the at least one blade includes a triangular cross-section. The needle may have a tip, located on the longitudinal axis of the needle and at least two, preferably three, blades tapering towards the tip. The needle may comprise at least one, preferably multiple barbs.

The drug may be liquid or solid. The drug may be loaded by soaking, spraying, or powdering the patch. For example, the patch is loaded by dipping the patch in a solution with the drug. In another example, the drug is distributed as a powder on the patch. Powdering may also include, solving the drug in a solution, and drying the solution on the patch. The fibers of the patch may be hydrophillic for a better loading.

In one embodiment, the patch comprises a first region and a second region, wherein the first region is loaded with the drug and the second region is not loaded with the drug of the first region. In particular, the regions extend along a surface of the patch. Thereby, the drug is only applied to certain region. In one example the outer edge of the patch may free of drugs. The amount of drug that is used may be reduced without impact on the treatment outcome. The regions may be visible for a user.

In a preferred embodiment, a first visible region comprises a marking. The first region may be loaded with a first drug and or the first region may be suitable to be felted to soft tissue. In a further alternative, the marking corresponds to a region that is to be felted in a particular application. For example, the present patches may be used for ligament repair. Based on the ligament that is repaired, different regions of the patch may need to be felted. Such regions can be marked so that a user can immediately see where the portions need to be felted, reducing the time needed for the operation. Further, such markings may help a user in identifying the correct orientation (e.g. top and bottom, up and down) of the patch during an operation, preventing errors.

Other applications, i.e. examples of soft tissue, of the present patch include the skin, tendons, menisci, spinal discs, fascia, skeletal muscle, heart muscle and valves, hollow organs (large vessels, bladder, oesophagus, intestine) and cartilage.

In a preferred embodiment, a second visible region is marked. The second region may be loaded with the second drug, and or maybe a region to be felted to soft tissue for a particular application.

In a preferred embodiment, the drug comprises one or more of the following:
mRNA, mRNA based drug, siRNA based drug, growth factors, verteporfin, genipin, analgesics, antacids, antianxiety drugs, antiarrhythmics, antibacterials, antibiotics, anticoagulants and thrombolytics, anticonvulsants, antidiarrheals, antiemetics, antifungals, antihistamines, antihypertensives, anti-inflammatories, antineoplastics, antipsychotics, antipyretics, antiviral, barbiturates, beta-blockers, bronchodilators, cold cures, corticosteroids, cough suppressants, cytotoxics, decongestants, diuretics, expectorant, hormones, hypoglycemics, immunosuppressives, laxatives, muscle relaxants, sedatives, sex hormones, sleeping drugs, tranquilizers, and vitamins.

In some embodiments, the patch may comprise more than one drug, e.g. two, three or more drugs. For example, a first drug may be an anit-inflammation drug and/or antibiotics and a second drug may be a drug that reduces scarring. The first drug may be initially released. The second drug may be released with a retardation.

Non-limiting embodiments of the invention are described, by way of example only, with respect to the accompanying drawings, in which:
- Figures 1A and 1B:: show a schematic illustration of an embodiment of the claimed invention, with a felting set comprising a felting device and an implant;
- Figures 2A to 2C:: show different embodiments of a drug loaded implant;
- Figure 3:: shows schematically how the implant having drug-loaded fibers and carrier fibers may release a drug to a soft tissue;
- Figure 4:: shows schematically how the implant of figure 2C may release a drug to a soft tissue;
- Figure 5:: shows schematically a further method of drug delivery;
- Figure 6:: shows a further embodiment of an implant according to the invention;
- Figures 7A to 7D:: show a embodiments of implants comprising a marking;
- Figures 8A to 8D:: show embodiments of a needle that can be used in the present invention;
- Figures 9A and 9B:: show processes for applying a drug to the implant.

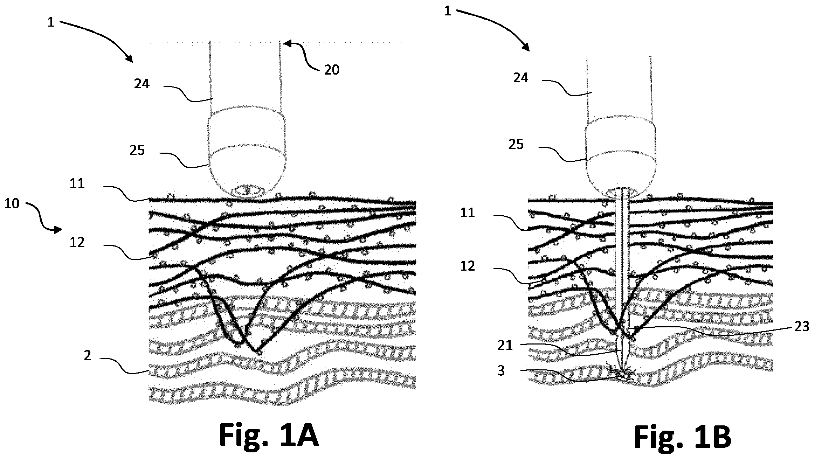

Figures 1A and 1B show a schematic illustration of an embodiment of the claimed invention, with a felting set 1 comprising a felting device 20 and an implant 10. The felting device 20 is shown only in part. In particular the felting device 20 may be the felting device as disclosed in any of the following applications: European Patent Application 21211467.2, PCT/EP2020/081887, WO 2020/227838 or PCT/EP2020/081891. The felting devices in PCT/EP2020/081887 are preferred.

The felting device 20 includes a hollow tube 24 with a tip 25. A felting needle 21 with multiple barbs 23 (see figure 1B) is arranged within the hollow tube 24. The felting device comprises a motor (not shown) that moves the felting needle 21 backwards and forwards between the positions shown in figure 1A and figure 1 B. The implant 10 comprises a patch that is made of individual fibers 11. The fibres 11 may be made of any of the materials disclosed in the applications above and have a diameter of 8 µm. A drug, such as an anti-inflammatory drug is attached to the individual fibers 11 of the implant 10. The implant 10 is laid on to a soft tissue 2. The soft tissue 2 may be a ligament. Other examples of soft tissue are mentioned above. The soft tissue also comprises individual fibers as is schematically shown in figures 1A and 1B.

When the felting needle 21 is moved forwards and out of the tube 24, the barbs 23 of the felting needle 21 catch individual fibers and drag the fibers with the drugs attached to it into the soft tissue. Then, the felting needle 21 is retracted again into the position shown in figure 1A. This process is repeated with a frequency of 1-200 Hz, preferably 10-100 Hz, most preferably 20-80 Hz and in one embodiment 40 Hz.

This process is repeated as described in the applications mentioned above. Thereby, the fibers of the patch are entangled with the fibers of the soft tissue providing a strong connection. The additional provision of an, e.g. anti-inflammatory, drug provides for a better response by the body of a patient.

The needle 21 causes lesions (micro-lesions 3) in the soft tissue (schematically shown in figure 1B). The microlesion may trigger a body response and improve the healing process.

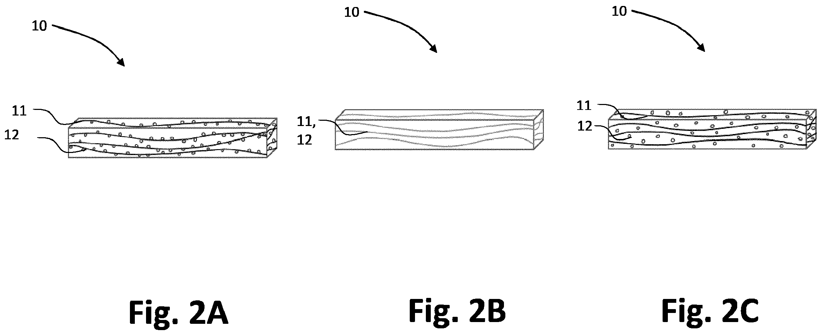

The drug may be incorporated into the implants 10 in different ways as can be seen from figures 2A to figure 2C. As shown in figure 2A (and also shown in figures 1A and 1B) the drug 12 may be dispersed and directly attached to the individual fibers 11 of the patch

Alternatively (as shown in figure 2B), the fibers 11 of the patch may form a hybrid structure comprising the drug 12. For example, the hybrid structure may be a PGA-drug conjugation. In a third example (see figure 2C), the drug is held in the space in between the fibres 11.

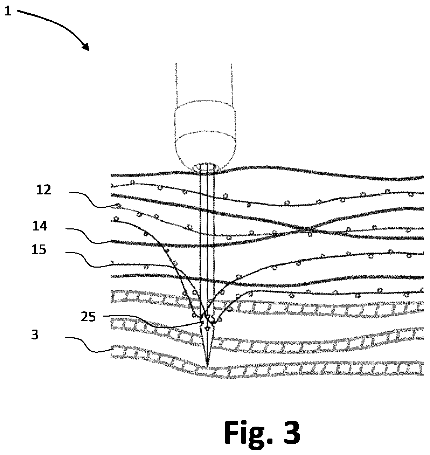

Figure 3 shows a schematic illustration of a further embodiment of the claimed invention. The embodiment may be similar to the embodiment shown in figures 1A and 1B. However, in the embodiment of figure 3, the implant comprises two types of fibres. First the implant comprises drug-loaded fibres 15 and carrier fibres 14. The drug-loaded fibers may have a diameter of 6 µm and the carrier fibres may have a size of 20 µm. As can be seen, the carrier fibres are much large than the drug-loaded fibres. Accordingly, an appropriatly shaped barb (e.g. having a size of 7µm) will catch only the drug-loaded fibres 14 while omitting the the carrier fibres which will not be felted to the soft tissue and thus provide stability.

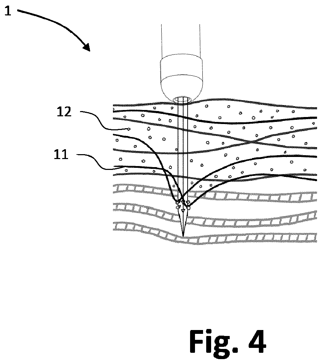

A further variation is shown in figure 4. As can be seen, the implant 10 is similar to the implant shown in figure 2C, in which the drug is held in the space in between the fibres 11. The drug is liquid. When such an implant is felted, as shown in figure 4, the drug is pushed with the fibres into the soft tissue. In some embodiments, the needle 21 may be hydrophillic or coated with a hydrophillic material such as Polyvinylpyrrolidon (PVP). The needle 21 may then push the drug into the fibres of the soft tissue.

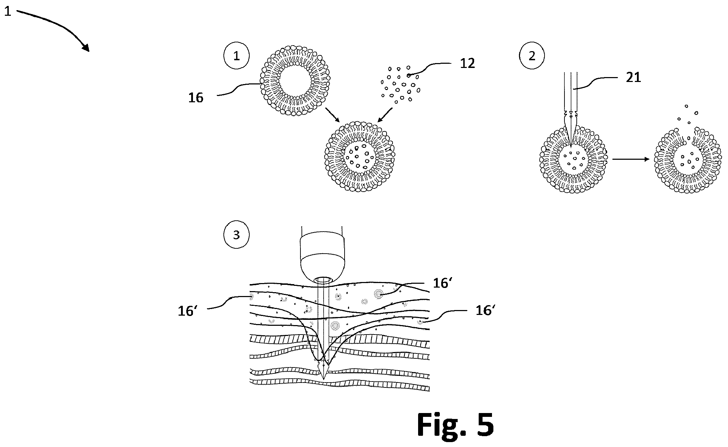

In a variation of the embodiment shown in figure 4, the drug may be carried by a liposome or micelle. Figure 5 shows an embodiment with a liposome 16. A liposome may be understood as a spherical vesicle having at least one lipid bilayer. The liposome 16 is be loaded with the drug and by used as a drug carrier. With respect to the present invention, liposomes may be particularly advantageous, as the needles 21 shown herein may be suitable to pierce the liposome and to release the drug contained therein. Thereby, the drug is precisely released when needed, e.g. during implantation. Figure 5 shows, that the drug 12 is loaded into a liposome 16 (step 1). The drug may then be released by piercing the lioposome 16 with the needle 21. Step 2 of figure 5 then shows how this implant would behave in use. When the needle 21 is moved back and forth, the the liposomes 16 are pierced (see liposomes 16' in figure 5). In other embodiments, the movement of the needle 21 alone may generate sufficient shear stress such that the drug is released.

The manufacture of liposomes and drug-loading of liposomes as such is known (see e.g. Shah S, Dhawan V, Holm R, Nagarsenker MS, Perrie Y. Liposomes: Advancements and innovation in the manufacturing process. Adv Drug Deliv Rev. 2020;154-155:102-122. doi: 10.1016/j.addr.2020.07.002. Epub 2020 Jul 8. PMID: 32650041).

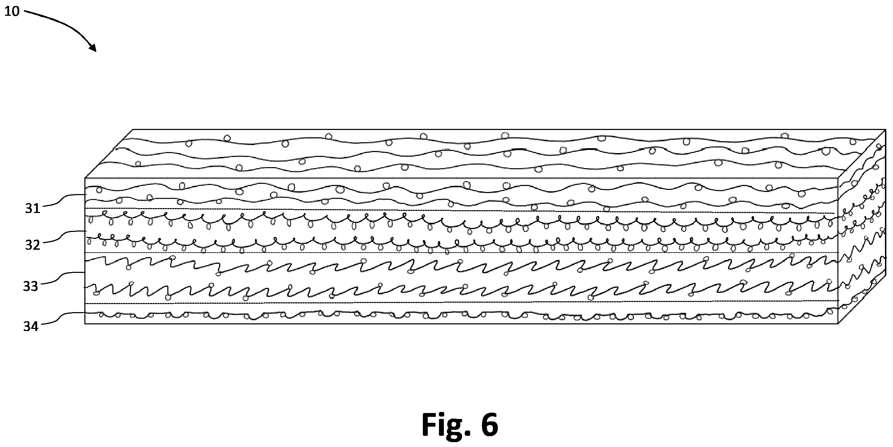

A further embodiment of an implant 10 is shown in figure 6. Figure 6 shows an implant with four layers 31, 32, 33 and 34. The layer shown at the lower end is intended to be put on the soft tissue of a patient. The layers have different degredation rates and drug-releasing speeds.

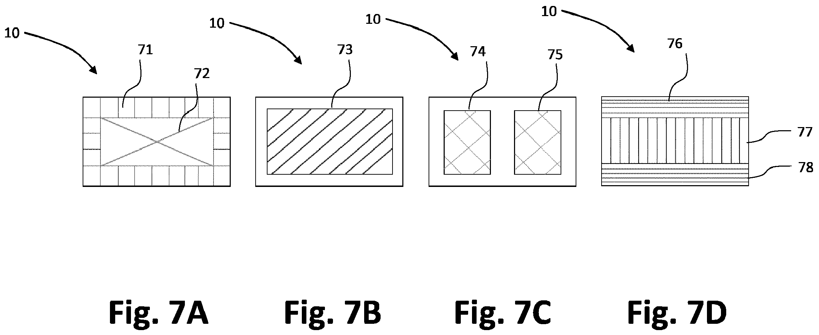

Figures 7A to 7D show a further embodiments of implants. Figures 7A to 7D show a top view of the implants comprising different markings. The markings are visible to a user. The markings may be a coloring, a pattern, an outline. For example, a stitching may be used. The markings may indicate to user where and if a certain drug is located.

This can be seen in figure 7A. A first region 71 (indicated by rectangular boxes) comprises a first drug and a second region 72 (indicated by a cross) comprises a second drug. The first region surrounds the second region. The regions 71, and 72 are shown as having a rectangular shape but may also have other shapes such as ovals, circles, triangles. In particular the regions may be shaped according to the anatomy of a soft tissue of a patient. For example, the second drug in the second region may include a growth factor for cartilage and may be shaped such that the second region is in contact with the cartilage when felted.

A second example is shown in figure 7B. The implant 10 in figure 7B has a marked first region (hatched in figure 7B). The marked region indicates the region of the implant 10 that can be felted. Thereby, a user can be prevented from felting regions that may provide poor attachment. For example, as shown in figure 7B, the outer edges may provide poor attachment, since the ends of the fibres are more likely to be drawn into the soft tissue which may lead to a less strong connection.

A third example of an implant 10 is shown in figure 7C. The implant 10 in figure 7C includes a first region 74 and a second region 75. The regions 74 and 75 are marked, such that a user knows where the implant 10 should suitably be felted.

A fourth example of markings on implant 10 is shown in figure 7D. The implant 10 in figure 7D comprises a first region 76, a second region 77 and a third region 78. Each of the regions is marked. This is indicated by the horizontal and vertical stripes in figure 7D. Each of the regions may have different properties indicated by the markings. For example the second region 77 may be biodegradable or may include a drug. The first and third regions 76 and 78 may have similar properties.

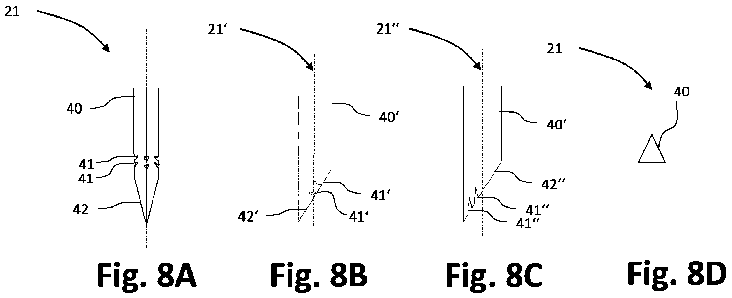

Figures 8A and 8D shows different preferred embodiments of the needle 21 of the felting device 1. As seen in figure 8A, the felting needle comprises a shaft portion 40 and a tip portion 42. The shaft and/or the tip portions 40, 42 may have a circular cross-section. In a preferred embodiment, the shaft and/or the tip portions 40, 42 may have the triangular cross-section shown in figure 8D. The tip portion 42 tapers. In the embodiment of figure 8A, the tip is conically shaped and tapers towards the center axis of the needle. Further, the felting needle 21 comprises six barbs 41. The barbs 41 are arranged at the shaft portion.

An alternative embodiment is shown in figure 8B. Figure 8B shows a needle 21' which comprises a shaft portion 40', a tip portion 42' and barbs 41'. In contrast to the embodiment of figure 8A, the barbs 41' are arranged in the tip portion 42'. Thus the barbs are arranged closer to the distal tip than in needle 21. As a result, a penetration depth of the needle may be reduced while achieving the same entanglement of tissue and patch fibres. Further, this reduces tissue damage caused by the felting needle 21.

In contrast to the tip portion of needle 21, the tip portion of needle 21' tapers towards an outer edge rather than the center axis. The tapering may form a one sided edge. The edge may be straight or curved. Further, the barbs may be formed by slits in the edge. The slits may be parallel (see figure 8C) or perpendicular (see figure 8B) (or any direction in between) to the longitudinal axis of the the needle 21'. Substantially parallel slits and the resulting barbs are preferred since the fibers may be caught more efficiently.

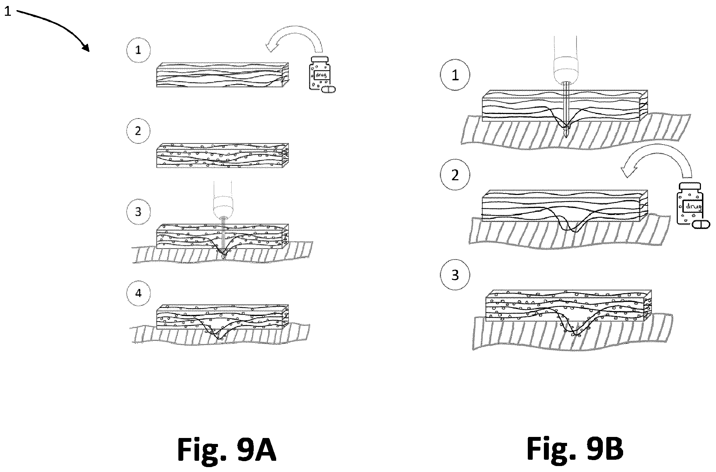

Typically the implant 10 is loaded with the drug while manufacturing the implant and in advance of an operation. This is shown in an examplatory manner in the process scheme of figure 9A. First, the drug is added to the implant as decribed above (step 1), then the prepared implant is applied to soft tissue (steps 2 and 3) and then the implant is felted to the soft tisse (steps 3 and 4). However, in some cases drugs may have a short life span or may need to be delivered seperatly for other reasons. In such cases, the order may be changed, as can be seen in figure 9B. In such embodiments the implant is first felted to the soft tissue. After the implant is (at least partially) felted to the tissue, the drug, in particular a liquid drug, is added, e.g. poured over the implant or a part of the implant. The drug is then distributed over the implant, e.g. through capillary forces. Since at least some of the fibres have been felted into the soft tissue, the drug may also be distributed to the soft tissue. In some embodiments, the implant may be felted once more in order to improve the distribution of the drug.

## Claims

1. A felting set (1) for attaching an implant (10) to soft tissue (2), comprising a felting device (20) and the implant, wherein the implant comprises a patch comprising a non-woven material made of individual fibers (11), wherein the fibers have a length of 20 to 80 mm, wherein the patch comprises a drug (12); and wherein the felting device includes a needle (21) with at least one blade (22) and with at least one barb (23; 24) for delivering the fibers of the patch into the soft tissue.

2. Felting set according to claim 1, wherein the drug (12) is attached to the fibers (11) or part of fibers.

3. Felting set according to the previous claim, wherein the fibers are polymer fibers and comprise a hybrid structure of drugs and polymer.

4. Felting set according to any one of the previous claims, wherein the patch comprises a first layer (31) and a second layer (32), wherein the first and second layers differ in degradation rate, drug releasing speed, thickness and/or strength.

5. Felting set according to any one of the previous claims, wherein the patch comprises a first and a second layer, wherein the first and second layer each comprise a drug and wherein the drugs of the first and second layers have different degradation rates.

6. Felting set according to any one of the previous claims, wherein the needle tip is at a radially outer edge (14) and the blade (22) extends across a cross-section of needle to an opposing other radially outer edge.

7. Felting set according to any one of the previous claims, wherein the drug is held in the space(13) in between the fibers (11).

8. Felting set according to any one of the previous claims, wherein the patch comprises carrier fibres (14) and drug-loaded fibres (15).

9. Felting set according to the previous claim, wherein the carrier fibres have a larger diameter thanthe drug-loaded fibres such that the at least one barb felts only the drug-loaded fibers into the soft tissue.

10. Felting set according to any one of the previous claims, wherein the patch comprises liposomes (16) or micelles, wherein the liposomes or micelles carry the drug.

11. Felting set according to the previous claims, wherein the needle is shaped such that the liposomes or micelles are damaged such that the drug is released by the needle while felting the patch.

12. Felting set according to the previous claims, wherein the drug is held by a carrier, in particular nanocapsules or nanospheres, wherein the carrier is attached to the fibres of the patch.

13. Felting set according to any one of the previous claims, wherein the drug comprises at one or more of: mRNA, mRNA based drug, siRNA based drug, growth factors, verteporfin, genipin, analgesics, antacids, antianxiety drugs, antiarrhythmics, antibacterials, antibiotics, anticoagulants and thrombolytics, anticonvulsants, antidiarrheals, antiemetics, antifungals, antihistamines, antihypertensives, anti-inflammatories, antineoplastics, antipsychotics, antipyretics, antiviral, barbiturates, beta-blockers, bronchodilators, cold cures, corticosteroids, cough suppressants, cytotoxics, decongestants, diuretics, expectorant, hormones, hypoglycemics, immunosuppressives, laxatives, muscle relaxants, sedatives, sex hormones, sleeping drugs, tranquilizers, and vitamins.

14. Felting set according to any one of the previous claims, wherein the needle comprises at least one barb (23) and the at least one barb (23) has a depth of 0.005-0.05 mm.

15. Felting set according to any one of the previous claims, wherein the largest section of the blade has a diameter of 0.1 to 2 mm, preferably 0.1 to 1 mm, particularly preferred 0.1 to 0.5 mm.

16. Felting set according to any one of the previous claims, wherein the drug is liquid or solid and preferably loaded by soaking, spraying, or powdering the patch.

17. Felting set according to any one of the previous claims, wherein the patch comprises a first region (33) and a second region (34), wherein the first region is loaded with the drug and the second region is not loaded with the drug of the first region.

18. Felting set according to any one of the previous claims, wherein a first visible region is marked, the first region being loaded with a first drug and/or being a region suitable to be felted to soft tissue, or being a region to be felted for a particular application.

19. Felting set according to any one of the previous claims, wherein a second visible region is marked, the second region being loaded with a second drug and/or being a region to be felted to soft tissue for a particular application.
